(19) Europäisches Patentamt
European Patent Office
Office européen des brevets

(11) Veröffentlichungsnummer: **0 307 786**
**A2**

# (12) EUROPÄISCHE PATENTANMELDUNG

(21) Anmeldenummer: **88114630.2**

(22) Anmeldetag: **07.09.88**

(51) Int. Cl.⁴: **C07B 59/00 , C07C 172/00 , A61K 31/59 , //C07C69/66, C07C35/32,C07F7/18**

(30) Priorität: **14.09.87 US 96981**

(43) Veröffentlichungstag der Anmeldung: **22.03.89 Patentblatt 89/12**

(84) Benannte Vertragsstaaten: **AT BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Baggiolini, Enrico Giuseppe**
**30 Evergreen Drive**
**North Caldwell, N.J. 07006(US)**
Erfinder: **Hennessy, Bernard Michael**
**111 Passaic Avenue**
**Nutley, N.J. 07110(US)**
Erfinder: **Uskokovic, Milan Radoje**
**253 Highland Avenue**
**Upper Montclair, N.J. 07043(US)**

(74) Vertreter: **Lederer, Franz, Dr. et al**
**Patentanwält Dr. Franz Lederer Lucile Grahnstrasse 22**
**D-8000 München 80(DE)**

## (54) Deutero-Cholecalciferolderivate, ihre Herstellung und pharmazeutische Anwendung.

(57) 26,26,26,27,27,27-Hexadeutero -1α,25-dihydroxycholecalciferol und 24,24,26,26,26,27,27,27-Octadeutero -1α,25-dihydroxycholecalciferol können als Heilmittel, z.B. zur Behandlung von Osteoporose oder Psoriasis dienen.

EP 0 307 786 A2

## Deutero-Cholecalciferolderivate ihre Herstellung und pharmazeutische Anwendung

Die Erfindung betrifft 26,26,26,27,27,27-Hexadeutero-1α,25-dihydroxycholecalciferol und 24,24,26,26,26,27,27,27-Octadeutero -1α,25-dihydroxycholecalciferol (im folgenden als die Hexadeutero- bzw. die Octadeuteroverbindung bezeichnet) als pharmazeutisch wirksame Substanzen, besonders zur Behandlung von Osteoporose oder entzündlichen Hautkrankheiten, insbesondee Psoriasis. Die Erfindung betrifft weiterhin Medikamente, die eine oder beide der erwähnten Verbindungen oder Gemische davon enthalten und ein Verfahren zur Herstellung dieser Medikamente sowie die Verwendung dieser Verbindungen zur Behandlung von Krankheiten oder zur Herstellung von Medikamenten zur Behandlung von Krankheiten, besonders zur Behandlung von Osteoporose oder entzündlicher Hauterkrankungen, insbesondere Psoriasis. Die Erfindung betrifft weiterhin die Octadeuteroverbindung, ein Verfahren zu deren Herstellung und die Herstellung der Hexadeuteroverbindung und Zwischenprodukte die in diesem Verfahren auftreten.

Dieses Verfahren ist dadurch gekennzeichnet, dass man eine Verbindung der Formel

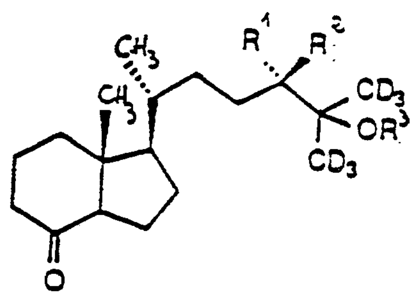

II

worin $R^1$ und $R^2$ beide Wasserstoff oder beide Deuterium und $R^3$ $-Si(\text{nieder-Alkyl})_3$ ist, mit einer Verbindung der Formel

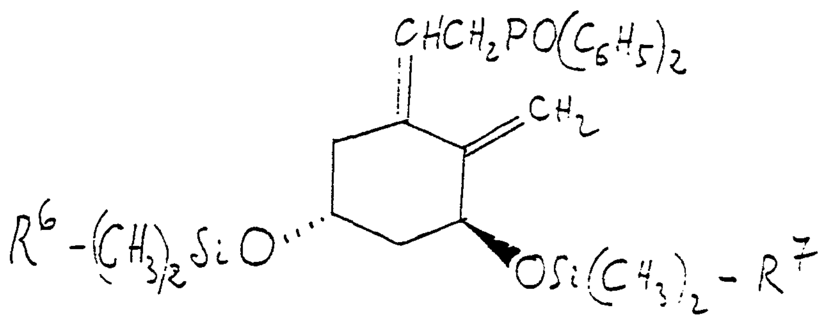

X

worin $R^6$ und $R^7$ nieder-Alkyl oder Aryl sind, umsetzt und aus der erhaltenen Verbindung die Schutzgruppen abspaltet.

Der hier verwendete Ausdruck "nieder-Alkyl" bezeichnet geradkettige oder verzweigte Gruppen mit bis zu 8 C-Atomen wie Methyl, Aethyl, Propyl oder t-Butyl; Aryl bedeutet Phenyl oder durch ein oder mehrere Alkyl-, Halogen-, Nitro-, Cyano-oder Trifluormethylgruppen substituiertes Phenyl.

Die obige Reaktion kann in Gegenwart einer Base wie nieder-Alkyllithium, z.B. Butyllithium oder mono- oder di-alkylierten Disilylamiden, in einem Aether wie Tetrahydrofuran (THF) unter Inertgasatmosphäre, z.B. Argon bei einer Temperatur im Bereich von etwa -80 bis -50 °C durchgeführt werden. Die erhaltene Verbindung kann durch Chromatographie an Silicagel gereinigt werden.

Die Entfernung der Schutzgruppen kann durch Behandlung mit einem niederen Alkanol oder mit einem Gemisch von Wasser, einem organischen Lösungsmittel und einer Säure bewerkstelligt werden. Beispiele von Säuren sind Mineralsäuren, nieder-Alkancarbonsäuren oder Sulfonsäuren oder, vorzugsweise, ein Kationenaustauscherharz in der H-Ionenform als Suspension in einem niederen Alkanol. Alternativ kann die Abspaltung der Schutzgruppe durch Behandlung mit Tetrabutylammoniumfluorid in einem Aether, z.B. THF durchgeführt werden.

Die Verbindungen der Formel II können über die Verbindungen der Formel III bis VIII und II′ wie auf der nächsten Seite dargestellt, hergestellt werden, wobei R nieder-Alkyl, Aryloder Aryl-nieder-alkyl, X Chlor oder Brom, Y Chlor, Brom, Jod oder $OSO_2R$, $R^{31}$ Wasserstoff oder -Si(nieder-alkyl)$_3$, $R^4$ entweder Wasserstoff und $R^5$ Wasserstoff oder $CO_2R$ oder $R^4$ -Si(nieder-alkyl)$_3$ und $R^5$ Wasserstoff bedeuten; und worin $R^1$ und $R^2$ die obige Bedeutung haben.

Formeln

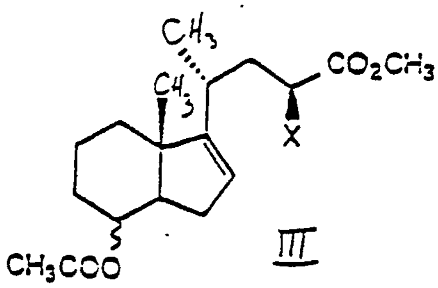

III

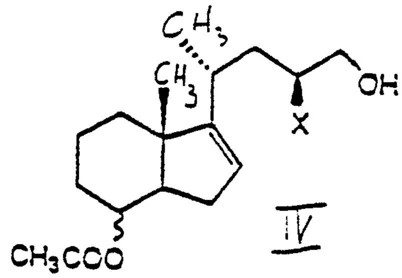

IV

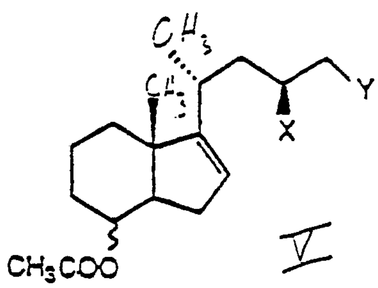

V

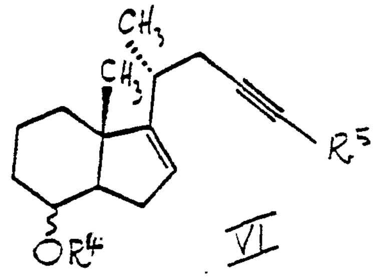

VI

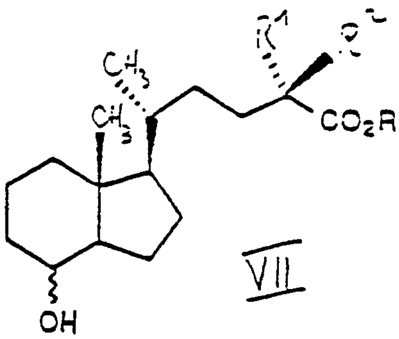

VII

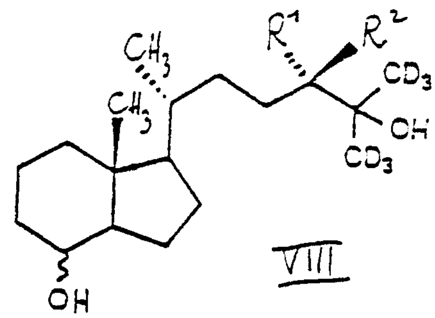

VIII

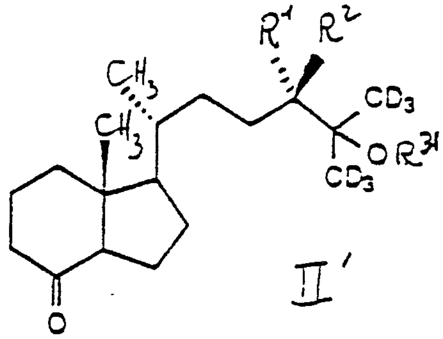

II′

Der Ester III wird zum Alkohol IV mit einem Hydrid, z.B. $LiAlH_4$ in einem Lösungsmittel wie einem aliphatischen oder aromatischen Kohlenwasserstoff, z.B. Hexan, Benzol oder Chloroform, einem Aether, z.B. THF bei Temperaturen im Bereich von -70 bis +80 °C, vorzugsweise bei etwa 0 °C bis Raumtemperatur reduziert.

Der Alkohol IV wird zunächst, z.B. durch Reaktion mit p-Toluolsulfonylchlorid in Dichlormethan und Pyridin in eine Verbindung der Formel V übergeführt, worin Y $OSO_2R$ ist. Diese Verbindung wird dann halogeniert, z.B. mit LiBr in einem polaren Lösungsmittel, z.B. Dimethylformamid (DMF) bei einer Temperatur im Bereich von 25-100 °C.

Das Halogenid V, worin Y Chlor, Brom oder Jod ist, wird in einen Alkohol der Formel VI, worin $R^4$ und $R^5$ Wasserstoff sind, durch Umsetzung mit einer starken Base, z.B. Natriumamid in t-Butylalkohol in einem polaren Lösungsmittel, z.B. Hexamethylphosphoramid bei einer Temperatur im Bereich von -20 bis +30 °C übergeführt.

Der Alkohol VI wird dann silyliert, z.B. mit 1-(Trimethylsilyl)imidazol in einem Aether, z.B. THF, bei Temperaturen im Bereich von etwa -20 bis +30 °C.

Der resultierende Aether VI, worin $R^4$ -Si(nieder-alkyl)$_3$ und $R^5$ Wasserstoff ist, wird zuerst mit einer starken Base, z.B. Butyllithium in einem aprotischen Lösungsmittel, z.B. Hexan, bei einer Temperatur im Bereich von -100 °C bis Raumtemperatur umgesetzt, dann mit einem Chloroformat $ClCO_2R$, worin R die obige Bedeutung hat, z.B. mit Methylchloroformat..

Der erhaltene Ester VI, worin $R^4$ Wasserstoff und $R^5$ $CO_2R$ und R die obige Bedeutung hat, wird dann in Gegenwart eines Katalysators, z.B. 5-10% Palladium auf Kohle in einem Lösungsmittel, wie einem Aether, einem aromatischen Kohlen wasserstoff, einem Alkancarbonsäure-niederalkylester oder einem niederen Alkanol, z.B. Aethanol, hydriert.

Der erhaltene gesättigte Ester VII, worin $R^1$ und $R^2$ Wasserstoff sind, kann deuteriert werden, z.B. mit Methyl-$d_3$-Alkohol-d in Gegenwart von Natriummethoxid-$d_3$ unter Inertgasatmosphäre, z.B. Argon, bei Temperaturen im Bereich von 0-80 °C, wobei ein Ester der Formel VII erhalten wird, worin $R^1$ und $R^2$ Deuterium sind.

Der Ester VII kann mit Methyllithium-$d_3$ in einem Aether, z.B. Diäthyläther, bei einer Temperatur im Bereich von -30 bis +30 °C unter Inertgasatmosphäre, z.B. Argon, umgesetzt werden.

In der nächsten Stufe wird das Diol VIII mit einem Oxidationsmittel, z.B. Pyridiniumchlorochromat in einem nieder-Alkylhalogenid-Lösungsmittel, z.B. Methylenchlorid, bei einer Temperatur im Bereich von -10 bis +30 °C umgesetzt.

Ein erhaltenes Keton II′, worin $R^{31}$ Wasserstoff ist, wird mit einem Silylierungsmittel in einem Lösungsmittel wie einem halogenierten Kohlenwasserstoff, z.B. Methylenchlorid, umgesetzt, wobei eine Verbindung der Formel II erhalten wird. Die Ausgangsmaterialien der Formel III und X sind bekannt oder können nach bekannten Verfahren wie in J. Org. Chem. 48, 1983, 4433 bzw. 51, 1986, 3098, hergestellt werden.

Die Zwischenprodukte der Formel II′, insbesondere

[1R-[1α(R*), 3aβ,7aα]]-Octahydro-1-[5-[trimethylsilyl)oxy]-1-methyl-5-(methyl-$d_3$)-6,6,6-$d_3$-hexyl]-7a-methyl-4H-inden-4-on und
[1R-[1α(R*), 3aβ, 7aα]]-Octahydro-1-[5-[trimethylsilyl)oxy]-1-methyl-5-(methyl-$d_3$)-4,4,6,6,6-$d_5$-hexyl]-7a-methyl-4H-inden-4-on,

die Zwischenprodukte der Formel VII, insbesondere

[1R-[1α(1R*),3aβ,4β,7aα]]-Octahydro-α,α-$d_2$-δ,7a-dimethyl-4-hydroxy-1H-inden-1-pentancarbonsäuremethylester und
[1R-[1α(1R*),3aβ,4β,7aα]]-Octahydro-δ-7a,-dimethyl-4-hydroxy-1H-inden-1-pentancarbonsäure-methyl ester,

die Zwischenprodukte der Formel VI, insbesondere

[3aS-[3(R*),3aα,7β]]-3a,4,5,6,7,7a-Hexahydro-3a-methyl-3-(1-methyl-3-butynyl)-1H-inden-7-ol,
[3aS-[3(R*),3aα,7β,7aβ]]-3a,4,5,6,7,7a-Hexahydro-3a-methyl-3-(1-methyl-3-butynyl)-7-[(trimethylsilyl)oxy]-1H-inden und
[3aS-[3(R*),3aα,7β,7aβ]]-5-(3a,4,5,6,7,7a-Hexahydro-7-hydroxy-3a-methyl-1H-inden-3-yl)-5-methyl-2-pentyncarbonsäure-methyl ester

sind neu und als solche Gegenstand der Erfindung.

Die wertvolle Aktivität der erfindungsgemässen deuterierten Verbindungen kann mit bekannten Verfahren, z.B. wie folgt gezeigt werden:

Die Stimulierung des Wachstums von Knorpelgewebe von Vögeln und Schweinen in vitro wurde durch Ermittlung der Syntheserate von Collagen und Proteoglycan ermittelt, die wiederum durch Zählen von [$^3$H]-Prolin, [$^3$H]-Thymidin und $^{35}SO_4$ in markiertem Knorpel ermittelt wurde. Die Resultate sind in der Tabelle I dargestellt im Vergleich zu 1$\alpha$,25-Dihydroxycholecalciferol (im folgenden 1$\alpha$,25$(OH)_2D_3$). Sie zeigen, dass die deuterierten Verbindungen Knorpelbildung stimulieren und somit die Bildung von neuem Knochen.

Die Wirkung von 1$\alpha$,25$(OH)_2D_3$ und Hexadeuteroverbindung wurde in verschiedenen Parametern bei Vitamin D-Mangel-Ratten bestimmt. Die Resultate in Tabelle II zeigen, dass die Hexadeuteroverbindung stärker aktiv ist als 1$\alpha$,25$(OH)_2D_3$ bei der Vermehrung der Knochenmasse, wobei die Serum $Ca^{++}$-Werte zeigen, dass die Hexadeuteroverbindung weniger aktiv als 1$\alpha$,25$(OH)_2D_3$ ist was die Induzierung einer Hypercalcämie betrifft.

Die Stimulierung der intestinalen Calciumabsorption (ICA) und die Mobilisierung von Knochencalcium (BCM) bei der Ratte wurden für die erfindungsgemässen deuterierten Verbindungen bestimmt. Weiter wurde die kompetitive Bindung (CP) dieser Verbindungen, d.h. deren Fähigkeit für 1$\alpha$,25$(OH)_2D_3$ Rezeptorbindestellen zu konkurrenzieren, mit Rezeptoren bestimmt, die aus Eingeweiden von Hühnern, Ratten und Kälbern gewonnen wurden. Die Resultate sind in Prozentsätzen der Wirkung von 1$\alpha$,25$(OH)_2D_3$ in Tabelle III dargestellt, und zeigen, dass die deuterierten Verbindungen dem 1$\alpha$,25$(OH)_2D_3$ vergleichbar sind.

Tabelle I

| Verbindung | Konzentration | Trockengewicht | In vitro-Zunahme (%) von | | | | | |
|---|---|---|---|---|---|---|---|---|
| | | | Vogelknorpel | | | Schweineknorpel | | |
| | | | $^{3}H$ Thymidin | $^{3}H$ Prolin | $^{35}S$ | Trockengewicht | $^{3}H$ Prolin | $^{35}S$ |
| $1\alpha,25(OH)_2D_3$ | $10^{-9}$ | 31,8 | | | | 49 | 300 | 280 |
| | $10^{-10}$ | 27,2 | | 122 | 21,8 | 0 | 0 | 0 |
| | $10^{-11}$ | 24,1 | | 68,3 | 13,8 | 0 | 0 | 0 |
| | $10^{-12}$ | 22,6 | | 46,3 | 8,6 | | | |
| Hexadeutero-Verbindung | $10^{-9}$ | 35,3 | | 148,3 | 41,0 | | | |
| | $10^{-10}$ | 29,1 | 44,3 | 145,4 | 30,1 | 57,6 | 215,7 | 466,0 |
| | $10^{-11}$ | 29,8 | | 131,4 | 26,3 | 40,9 | 123,6 | 203,2 |
| | $10^{-12}$ | 31,2 | 65,2 | 65,0 | | 31,5 | | |
| | $10^{-13}$ | 20,5 | 57,9 | 36,0 | 21,5 | 29,5 | | |
| Octadeutero-Verbindung | $10^{-9}$ | 28 | | | 10,8 | 60 | 150 | 490 |
| | $10^{-10}$ | 21,6 | 25,0 | | | 50 | 75 | 410 |
| | $10^{-11}$ | 17,0 | 20,7 | | | 42 | 20 | 95 |
| | $10^{-12}$ | 10,3 | 15,3 | | | | | |

Tabelle II

| Verbindung | tägliche Dosis (mg) | Körpergewicht (g) | Konzentration (mg/dl) im Serum von: | | | Knochenmasse |
|---|---|---|---|---|---|---|
| | | | $Ca^{2+}$ | $PO_4^{2+}$ | Kreatinin | |
| 1α,25(OH)$_2$D$_3$ | 15<br>60 | 192±4,5<br>156±15 | 11,87±0,16<br>11,92±0,40 | 7,61±0,31<br>6,85±0,30 | 0,37±0,04<br>0,35±0,02 | 156±16<br>296±56 |
| Hexadeutero-Verbindung | 16,5<br>66 | 193±5<br>163±7,2 | 11,28±0,26<br>11,20±0,28 | 7,08±0,07<br>6,61±0,36 | 0,36±0,02<br>0,36±0,04 | 288±58<br>371±68 |

Tabelle III

| Verbindung | ICA | BCM | CP in den Eingeweiden von | | |
|---|---|---|---|---|---|
| | | | Ratte | Huhn | Kalb |
| Hexadeutero<br>Octadeutero | 92<br>106 | 64<br>147 | 100 | 106<br>104 | 100<br>76 |

Die Hexadeutero- und Octadeuteroverbindungen können in Dosierungen im Bereich von etwa 0,25 bis 2 µg pro Tag verabreicht werden zur Behandlung von Krankheitszuständen, wie Osteoporose und entzündlichen Hauterkrankungen, z.B. Psoriasis und Kontaktdermatitis. Sie können oral, subkutan, intramuskulär, intravenös oder intraperitoneal zur Behandlung von Osteoporose verabreicht werden und zur Behandlung von entzündlichen Hauterkrankungen auch topisch.

Die erfindungsgemässen deuterierten Verbindungen können in Präparaten wie Tabletten, Kapseln oder Elixiren zur oralen Verabreichung vorliegen, in sterilen Lösungen oder Suspensionen zur parenteralen Verabreichung oder in topischen Formulierungen. Etwa 0,25 bis 2 µg der Verbindung kann mit pharmazeutisch anwendbaren Hilfsstoffen beispielsweise Bindemitteln wie Tragacanth-Gummi, Akaziengummi, Maisstärke und Gelatine, Streckmitteln wie Dicalciumphosphat, Sprengmitteln wie Mais- oder Kartoffelstärke und Alginsäure, Schmiermitteln wie Magnesiumstearat, Süsstoffen wie Sucrose, Lactose oder Saccharin und Geschmacksstoffen wie Pefferminz und Kirscharoma verarbeitet werden. Tabletten können mit Shallac, Zucker oder beidem überzogen sein. Ein Sirup oder Elixir kann Methyl oder Propylhydroxybenzoat als Konservierungsmittel enthalten. Beispiele von Vehikeln für sterile Zusammensetzungen für Injektionen sind Wasser, natürlich vorkommende vegetabile Oele, z.B. Sesam-, Kokussnuss-, Erdnuss- oder Baumwollsamenöl oder synthetisches Aethyloleat. Weiterhin können Puffer, Konservierungsmittel und Antioxidantien eingearbeitet werden.

## Beispiel 1

1a) Eine Lösung von 1,01 g (2,95 mMol) [3aS-[3(αS*γR*),-3aα,7β,7aβ]-7-(Acetyloxy)-α-chlor-3a,4,5,6,7,7a-hexahydro-γ,3a-dimethyl-1H-inden-3-butansäure-methylester in 25 ml THF wurde auf 0°C gekühlt und unter Argon mit 1,47 ml (1,47 mMol) einer 1M-Lösung von $LiAlH_4$ in THF behandelt. Nach 2 Stunden Rühren bei 0°C wurde weitere 0,20 ml Lithiumaluminiumhydrid-Lösung zugesetzt und die Reaktion eine weitere Stunde ablaufen gelassen. Sie wurde dann durch sorgfäligen Zusatz von 8 ml einer 2N Kaliumnatriumtartratlösung gefolgt von 0,5 ml 2N Salzsäure abgebrochen. Nach Verdünnen mit Wasser wurde mit Aethylacetat extrahiert. Die vereinigten Extrakte wurden mit 2N Kaliumnatriumtartratlösung und Kochsalzlösung gewaschen, getrocknet und zur Trockene eingedampft. Man erhielt 0,90 g (93% Ausbeute) [3aS-[3-(βS*,-δR*),3aα,7β,7aβ]]-7-Acetoxy-β-chlor -3a,4,5,6,7,7a-hexahydro-δ,3α-dimethyl-1H-inden-3-butanol, $[\alpha]_D^{23}$ -30.1° (c = 0.6, Aethanol).

1b) Eine Lösung von 1,50 g (4,76 mMol) des Produkts von Beispiel 1a) in 20 ml Dichlormethan und 20 ml Pyridin wurde bei 0° unter Argon mit 4,50 g (23,60 mMol) p-Toluolsulfonylchlorid versetzt und dann über Nacht bei 0°C gerührt. Danach wurden 8 ml Wasser zugesetzt und nach Rühren des Gemisches während 1 Stunde wurden die Lösungsmittel unter vermindertem Druck entfernt und der Rückstand mit Wasser verdünnt und mit Aethylacetat extrahiert. Die organischen Extrakte wurden mit 1N Salzsäure, Wasser, 2N wässriger Kaliumbicarbonatlösung und Kochsalzlösung gewaschen und getrocknet. Der Rückstand wurde in 40 ml DMF gelöst und unter Argon mit 4,00 g (46,06 mMol) wasserfreiem LiBr 17 Stunden erwärmt. Nach Kühlen wurde das Reaktionsgemisch mit Eiswasser verdünnt und mit Aethylacetat extrahiert. Die vereinigten organischen Extrakte wurden mit Wasser gewaschen, getrocknet und zur Trockene eingedampft. Man erhielt 1,62 g (Gesamtausbeute 90%) [3aS-[3(1R*,3S*),-3aα,7β,7aβ]]-7-Acetoxy-3-(3-chlor -4-brom-1-methylbutyl)-3a,4,5,6,7,7a-hexahydro -3a-methyl-1H-inden, $^1H$ NMR (60 MHz, $CDCl_3$) δ0,88 (s, 3H), 1,07 (d, J = 6,4 Hz, 3H), 2,03 (s, 3H), 3,60 (br AB q, J = 10.2 Hz, Δγ = 14,0 Hz, 2H), 5,00 (br m, 1H), 5,33 (br s, 1H) ppm.

1c) Eine Suspension von 1,40 g (35,89 mMol) Natriumamid in 15 ml Hexamethylphosphoramid wurde zunächst unter Argon mit 1,10 ml (11,66 mMol) t-Butylalkohol während 5 Minuten versetzt, sodann mit einer Lösung von 1,31 g (3,46 mMol) des Produkts von 1b) in 20 ml Hexamethylphosphoramid. Das erhaltene Gemisch wurde 2,5 Stunden bei Raumtemperatur gerührt, danach wurde die Reaktion durch sorgfältigen Zusatz von eiskalter wässriger Ammonchloridlösung gefolgt von Ansäuern mit 2N HCl abgebrochen und es wurde mit Aethylacetat extrahiert. Die vereinigten organischen Phasen wurden mit gesättigter Ammoniumchloridlösung und danach mit Wasser gewaschen, getrocknet und unter vermindertem Druck eingedampft. Der Rückstand wurde durch Chromatographie an Silicagel (mit Hexan-Aethylacetat 2:1 als Lösungsmittel) gereinigt. Man erhielt 0.27 g (36% Ausbeute) [3aS-[3(R*),3aα,7β,7aβ]]-3a,4,5,6,7,7a-Hexahydro -3a-methyl-3-(1-methyl-3-butynyl)-1H-inden-7-ol, $[\alpha]_D^{25}$ -4.1° (c 0.5 in Aethanol).

## Beispiel 2

Eine Lösung von 0,680 g (3,11 mMol) des Produkts von Beispiel 1c) in 15 ml THF wurde mit 0,700 ml 1-(Trimethylsilyl)imidazol (4,77 mMol) versetzt und das erhaltene Gemisch wurde unter Argon 2 Stunden bei Raumtemperatur gerührt. Das Lösungsmittel wurde dann zur Trockene abgedampft und der Rückstand auf eine Silicagelsäule gegeben, die mit Hexan-Aethylacetat (10:1) eluiert wurde. Man erhielt 0,845 g (94% Ausbeute) [3aS-[3(R*),3aα,7β,7aβ]]-3a,4,5,6,7,7a-Hexahydro -3a-methyl-3-(1-methyl-3-butynyl) -7-[-(trimethylsilyl)oxy]-1H-inden als dickes Oel, das unmittelbar im nächsten Schritt eingesetzt wurde.

## Beispiel 3

Eine Lösung von 0,400 g (1,38 mMol) des Produkts von Beispiel 2 in 8 ml THF wurde tropfenweise bei -78°C und unter Argon mit 1,05 ml (1,68 mMol) einer 1,6M Lösung von Butyllitihium in Hexan versetzt. Nach 30 Minuten Rühren bei -78°C wurde die erhaltene Lösung unter Rühren in eine Lösung von 0,225 ml(2,91 mMol) Methylchloroformat in 2 ml THF, ebenfalls bei -78°C und unter Argon, gegeben. Danach liess man das Reaktionsgemisch im Verlaufe einer Stunde auf Raumtemperatur aufwärmen und rührte dann eine weitere Stunde. Das Reaktionsgemisch wurde dann langsam mit 5 ml einer 5%igen wässrigen Natriumdihydrogenphosphatlösung versetzt. 15 Minuten gerührt und mit Aethylacetat extrahiert. Die vereinigten organischen Phasen wurden mit 2N Salzsäure, Wasser und Kochsalzlösung gewaschen, getrocknet und zur Trockene eingedampft. Der Rückstand wurde durch Schnellchromatographie an Silicagel (Hexan-Aethylacetat 2:1 als Eluens) gereinigt und lieferte 0,348 g (91% Ausbeute) [3aS-[3(R*),3aα,7β,7aβ]]-5-(3a,4,5,6,7,7a-Hexahydro -7-hydroxy-3a-methyl-1H-inden-3-yl)-5-methyl -2-pentynsäure-methylester, $[\alpha]_D^{25}$ -7.1° (c 0.4, Aethanol).

## Beispiel 4

Eine Lösung von 0,300 g (1,09 mMol) des Produkts von Beispiel 3 in 20 ml Aethanol wurde mit 0,100 g 5% Palladium/Kohle-Katalysator versetzt und das Gemisch wurde über Nacht bei Atmosphärendruck hydriert. Abfiltrieren des Katalysators und Eindampfen des Lösungsmittels lieferte 0,294 g (96% Ausbeute) [1R-[1α(1R*),3aβ,4β,7aα]]-Octahydro-δ,7a-dimethyl -4-hydroxy-1H-inden-1-pentansäure-methyl ester, $[\alpha]_D^{25}$ +11.6 (c 0.2, Aethanol).

## Beispiel 5

5a) Eine ätherische Lösung von 2 ml (3,00 mMol) Methyllithium-$d_3$ wurde mit 6 ml Diäthyläther verdünnt und unter Argon bei 0°C tropfenweise mit 0,147 g (0,52 mMol) des Produktes von Beispiel 4, gelöst in 2,5 ml THF, versetzt. Nach 40 Minuten Rühren bei 0°C wurden weitere 0,5 ml (0,75 mMol) Methyllithium-$d_3$-Lösung zugesetzt und das Gemisch wurde weitere 25 Minuten reagieren gelassen. Danach wurde die Reaktion durch Zusatz mit einer Mischung von 2. ml 2N Kaliumcarbonat und 2 ml Kaliumnatriumtartrat-Lösung gestoppt, auf Raumtemperatur aufwärmen gelassen und mit Aethylacetat extrahiert. Die vereinigten organischen Extrakte wurden mit 2N Kaliumbicarbonat und danach mit Kochsalzlösung extrahiert, getrocknet und zur Trockene eingedampft. Der Rückstand wurde durch Schnellchromatographie über Silicagel (Hexan-Aethylacetat 1:1 als Eluens) gereinigt und lieferte 0.119 mg (79% Ausbeute) [1R-[1α(1R*)3aβ,4β,7aα]]-Octahydro-ε,7a-dimethyl -α,α-(dimethyl-$d_3$)-4-hydroxy-1H-inden-1-pentanol, $^1$H NMR (100 MHz, $CDCl_3$) δ 0,68 (s, 3H), 0.93 (d, J = 6,0 Hz, 3H), 3,57 (m, 1H) ppm.

5b) Eine Lösung von 0,117 g (0,406 mMol) des Produkts von Beispiel 5a) in 60 ml Methylenchlorid wurde bei Raumtemperatur 2,5 Stunden mit 0,270 g(1,253 mMol) Pyridiniumchlorochromat gerührt. Die erhaltene Suspension wurde mit Aether verdünnt, filtriert und der Rückstand mit Aether verrieben und filtriert. Die vereinigten Filtrate wurden zur Trockene eingedampft und der Rückstand durch Chromatographie an Silicagel (Eluens: Hexan-Aethylacetat 2:1) gereinigt. Das erhaltene Produkt (0,105 g) wurde in 6 ml Methylenchlorid gelöst, mit 0,225 ml (1,534 mMol) 1-(Trimethylsilyl)imidazol versetzt und 16 Stunden unter Argon bei Raumtemperatur gerührt. Danach wurden 2 ml Wasser zugesetzt und nach 20 Minuten Rühren wurde das erhaltene Gemisch mit Aethylacetat extrahiert. Die vereinigten Extrakte wurden mit Wasser und Kochsalzlösung gewaschen, getrocknet und zur Trockene einge dampft. Der Rückstand wurde durch Filtration über Silicagel und Elution mit Hexan-Aethylacetat (5:1) gereinigt und lieferte 0,120 g (82% Gesamtausbeute) [1R-[1α(1R*),-3aβ,7aα]]-Octahydro -1-[5-[(trimethylsilyl)oxy]-1-methyl-5-(methyl-$d_3$) -6,6,6-$d_3$-hexyl]-7a-methyl-4H-inden-4-on als farblose Flüssigkeit.

## Beispiel 6

Eine Lösung von 0,284 g (0,488 mMol) [3S-(1Z,5α,5β)] -[2[3,5-bis[[(1,1-Dimethylethyl)dimethylsilyl]oxy] -2-methylencyclohexyliden]äthyl]diphenylphosphinoxid in 8 ml THF wurde auf -78°C gekühlt und tropfenweise unter Argon mit 0,295 ml (0,472 mMol) einer 1,6M Lösung von Butyllithium in Hexan versetzt. Die erhaltene Lösung wurde 5 Minuten gerührt, dann tropfenweise bei -78°C mit einer Lösung von 0,120 g (0,334 mMol) des Produkts von Beispiel 5b) in 3 ml THF im Verlauf von 10 Minuten versetzt. Danach wurde weitere 2 Stunden unter Argon bei -78°C gerührt. Nach Zusatz von 3 ml eines 1:1-Gemisches von 2N Kaliumnatriumtartratlösung und 2N Kaliumbicarbonatlösung wurde das Reaktionsgemisch mit Aethylacetat bei Raumtemperatur extrahiert. Die organischen Extrakte wurden mit Kochsalzlösung gewaschen, getrocknet und zur Trockene eingedampft. Der Rückstand wurde durch Filtration über Silicagel (Eluens: Hexan-Aethylacetat 30:1) gereinigt und lieferte 0,217 g Produkt. Dieses wurde in einem Gemisch von 1 ml Methylenchlorid und 8 ml Methanol gelöst, mit 3,0 g Kationenaustauscherharz versetzt und die erhaltene Suspension wurde unter Argon bei Raumtemperatur 18 Stunden gerührt. Nach Filtrieren und Waschen des Harzes mit Methanol wurden die Filtrate zur Trockene eingedampft. Der Rückstand wurde durch Chromatographie an Silicagel (Elution mit Hexan-Aethylacetat 1:4) gereinigt und lieferte 0,112 g (79% Gesamtausbeute) 26,26,26,27,27,27-Hexadeutero -1α,25-dihydroxycholecalciferol, Smp. 117-118°C (nach Umkristallisation aus Methylformiat), $[\alpha]_D^{25}$ +43.3° (c 0.2, Aethanol).

## Beispiel 7

Eine Lösung von 0,288 g (1,020 mMol) des Produkts von Beispiel 5 in 5 ml Methyl-$d_3$-Alkohol-d wurde mit 0,1 ml einer 3N Lösung Natriummethoxid-$d_3$ in Methyl-$d_3$-Alkohol-d versetzt und das erhaltene Gemisch wurde unter Argon 9 Stunden zum Rückfluss erhitzt, dann über Nacht auf 50° C. Die vorhandene Base wurde dann durch Zusatz von Kationenaustauscherharz neutralisiert, wurde dann filtriert, das Harz mit Methanol gewaschen und die Filtrate zur Trockene eingedampft. Der Rückstand wurde durch Chromatographie an Silicagel (Elution mit Hexan-Aethylacetat 2:1) gereinigt. Das erhaltene Produkt (0,154 g) wurde in 5 ml Methyl-$d_3$-Alkohol-d in Gegenwart von 0,1 ml 3N Natriummethoxid-$d_3$ in Methyl-$d_3$-Alkohol-d gelöst, 4 Stunden zum Rückfluss erhitzt, aufgearbeitet und wie oben beschrieben gereinigt, wobei 0,066 g (223% Ausbeute) [1R[1α(1R*),3aα,4β,7aα]]-Octahydro-α,α -$d_3$-δ,7a-dimethyl-4-hydroxy-1H-inden -1-pentansäuremethylester als farbloses Oel erhalten wurden.

## Beispiel 8

Mittels des in den Beispielen 5a) und b) beschriebenen Verfahrens wurden 0,065 g des Produkts von Beispiel 7 umgewandelt in

a) 0,049 g (75% Ausbeute) [1R][1α(1R*),3aβ,4β,7aα]]-Octahydro-ε,7a-dimethyl-β,β-$d_2$-α,α-(dimethyl-$d_3$)-4-hydroxy-1H-inden-1-pentanol, weisser Feststoff und

b) 0,047 g dieser Verbindung wurde überführt in 0,051 g [1R-[1α(1R*),3aβ,7aα]]-Octahydro-1-[5-[-(trimethylsilyl)oxy]-1-methyl-5-(methyl-$d_3$)-4,4,6,6,6-$d_5$-hexyl]-7a-methyl-4H-inden-4-on, farbloses Oel.

## Beispiel 9

Mittels des in Beispiel 6 beschriebenen Verfahrens wurden 0.051 g des Produkts von Beispiel 8b) in 0,050 g (83% Gesamtausbeute) 24,24,26,26,26,27,27,27-Octadeutero -1α,25-dihydroxycholecalciferol, Smp. 104-107° C; Massenspektrum: 95,8% $d_8$, 2,2% $d_4$; 1.0% $d_3$ umgewandelt.

## Beispiel 10

Kapseln und eine Crème, die die Hexadeutero- oder Octadeuteroverbindung enthalten, können an sich bekannter Weise nach den folgenden Angaben hergestellt werden:

| | | mg pro Kapsel: | |
|---|---|---|---|
| a) | Hexadeutero- oder Octadeuteroverbindung | 0,000250 | 0,002 |
| | fraktioniertes Kokosöl | 199,995 | 199,990 |
| | butyliertes Hydroxyanisol | 0,01 | 0,01 |
| | Ascorbylpalmitat | 1,0 | 1,0 |
| | | g pro kg Crème: | |
| b) | Hexadeutero- oder Octadeuteroverbindung | 0,0001 | 0,01 |
| | Glycerylmonostearat | 100,00 | 100,00 |
| | Polysorbat | 20,00 | 20,00 |
| | Cetylalkohol | 50,00 | 50,00 |
| | Petrolatum | 70,00 | 70,00 |
| | Methylhydroxybenzoat | 1,50 | 1,50 |
| | Propylhydroxybenzoat | 0,50 | 0,50 |
| | Propyleneglykol | 200,00 | 200,00 |
| | gereinigtes Wasser | 568,05 | 568,05 |

**Ansprüche**

1. 26,26,26,27,27,27-Hexadeutero -1α,25-dihydroxycholecalciferol und 24,24,26,26,26,27,27,27-Octadeutero -1α,25-dihydroxycholecalciferol zur Anwendung als Heimittel.

2. Pharmazeutisches Präparat, enthaltend 26,26,26,27,27,27-Hexadeutero -1α,25-dihydroxycholecalciferol oder 24,24,26,26,26,26,27,27,27-Octadeutero -1α,25-dihydroxycholecalciferol oder Gemische davon.

3. Verwendung von 26,26,26,27,27,27-Hexadeutero -1α,25-dihydroxycholecalciferol oder 24,24,26,26,26,27,27,27-Octadeutero -1α,25-dihydroxycholecalciferol zur Herstellung von pharmazeutischen Präparaten zur Behandlung von Osteoporose und entzündlichen Hauterkrankungen, insbesondere Psoriasis.

4. 24,24,26,26,26,27,27,27-Octadeutero -1α,25-dihydroxycholecalciferol.

5. Eine Verbindung der Formel

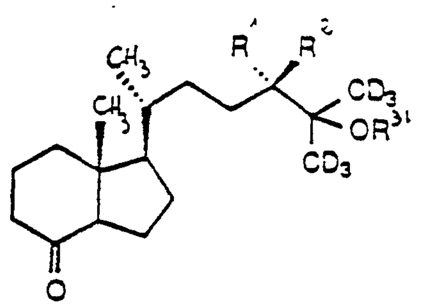

II'

worin $R^1$ und $R^2$ beide Wasserstoff oder Deuterium und $R^{31}$ Wasserstoff oder -Si(nieder-Alkyl)$_3$ sind.

6. [1R-[1α(R*), 3aβ,7aα]]-Octahydro-1-[5-[trimethylsilyl)oxy]-1-methyl-5-(methyl-d$_3$)-6,6,6-d$_3$-hexyl]-7a-methyl-4H-inden-4-on.

7. [1R-[1α(R*), 3aβ, 7aα]]-Octahydro-1-[5-[trimethylsilyl)oxy]-1-methyl-5-(methyl-d$_3$)-4,4,6,6,6-d$_5$-hexyl]-7a-methyl-4H-inden-4-on.

8. Eine Verbindung der Formel

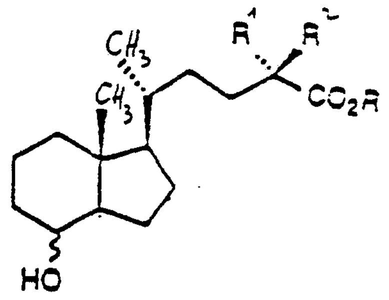

VII

worin $R^1$ und $R^2$ beide Wasserstoff oder Deuterium, und R nieder-Alkyl, Aryl oder Aryl-nieder-Alkyl sind.

9. [1R-[1α(1R*),3aβ,4β,7aα]]-Octahydro-α,α-$d_2$-δ,7a-dimethyl-4-hydroxy-1H-inden-1-pentansäuremethylester.

10. [1R-[1α(1R*),3aβ,4β,7aα]]-Octahydro-δ-7a-dimethyl-4-hydroxy-1H-inden-1-pentansäure-methylester.

11. Eine Verbindung der Formel

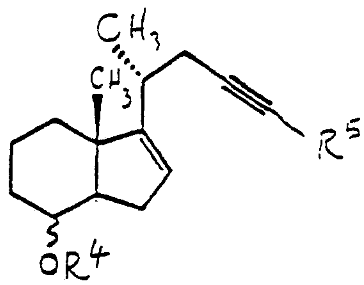

VI

worin $R^4$ entweder Wasserstoff und $R^5$ Wasserstoff oder $CO_2R$ ist, wobei R nieder-Aralkyl, Aryl oder Aryl-nieder-Alkyl ist, oder $R^4$ -Si(nieder-Alkyl)$_3$ und $R^5$ Wasserstoff ist.

12. [3aS-[3(R*),3aα,7β]]-3a,4,5,6,7,7a-Hexahydro-3a-methyl-3-(1-methyl-3-butinyl)-1H-inden-7-ol,
[3aS-[3(R*),3aα,7β,7aβ]]-3a,4,5,6,7,7a-Hexahydro-3a-methyl-3-(1-methyl-3-butinyl)-7-[(trimethylsiyl)oxy]-1H-inden und
[3aS-[3(R*),3aα,7β,7aβ]]-5-(3a,4,5,6,7,7a-Hexahydro-7-hydroxy-3a-methyl-1H-inden-3-yl)-5-methyl-2-pentinsäuremethylester.

13. Verfahren zur Herstellung von 26,26,26,27,27,27-Hexadeutero -1α,25-dihydroxycholecalciferol und 24,24,26,26,26,27,27,27-Octadeutero -1α,25-dihydroxycholecalciferol, dadurch gekennzeichnet, dass man eine Verbindung der Formel

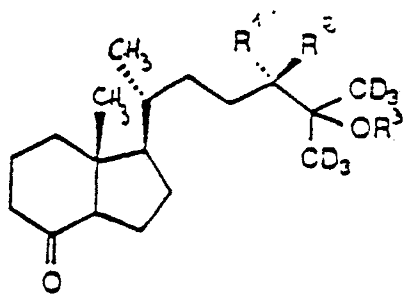

II

worin $R^1$ und $R^2$ beide Wasserstoff oder beide Deuterium und $R^3$ -Si(nieder-Alkyl)$_3$ ist, mit einer Verbindung der Formel

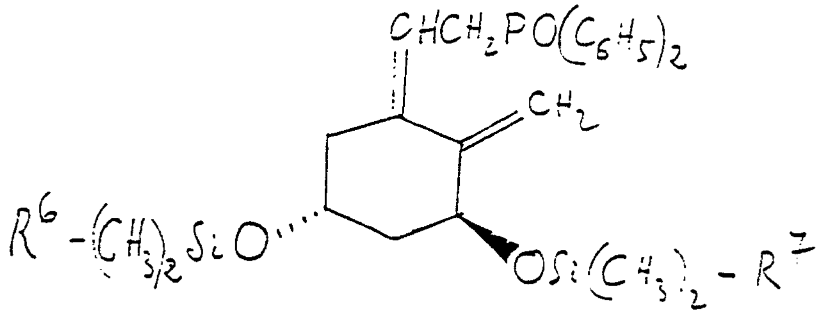

X

worin $R^6$ und $R^7$ nieder-Alkyl oder Aryl sind, umsetzt und aus der erhaltenen Verbindung die Schutzgruppen abspaltet.